# EUROPEAN PATENT SPECIFICATION

(11) **EP 1 656 162 B1**
(45) Date of publication and mention of the grant of the patent: **20.12.2006**
(21) Application number: 04753468.0
(22) Date of filing: 26.05.2004
(51) Int. Cl.: A61L 15/24, A61L 15/26, A61L 15/28, A61L 26/00

(54) **ABSORBENT CORES FOR ABSORBENT ARTICLES AND METHOD FOR MAKING SAME**
ABSORBTIONSKERNE FÜR ABSORBIERENDEN ARTIKEL UND DEREN HERSTELLUNGSVERFAHREN
NOYAUX ABSORBANTS DESTINES A DES ARTICLES ABSORBANTS ET LEUR PROCEDE DE PRODUCTION

(30) Priority: 20.08.2003 US 496720 P
(43) Date of publication of application: 17.05.2006
(73) Proprietor: Tyco Healthcare Retail Services AG, 8201 Schaffhausen (CH)
(72) Inventor: GLAUG, Frank, Chester Springs, PA 19425 (US); COLE, Robert, Jackson, NJ 08527 (US)
(74) Representative: Hermann, Bettina Celia
(86) International application number: PCT/US2004/016641
(87) International publication number: WO 2005/018694

(56) References cited:
- EP-A- 0 763 364
- EP-A- 1 013 291
- WO-A-01/91685
- US-A- 4 944 963
- US-A- 5 593 399
- US-A- 5 641 561
- US-B1- 6 417 425

## Description

This invention relates to absorbent articles that contain superabsorbent polymers, and the methods of making such articles. More particularly, this invention relates to absorbent cores used in absorbent articles.

### BACKGROUND OF THE INVENTION

Currently available absorbent articles include tissues that contain superabsorbent polymers (SAP), and air laid composites containing SAP particles and/or superabsorbent fibers. Traditional methods of absorbent article manufacture use SAP applied as a solid particulate material. Although the SAP particles in such absorbent articles afford the advantage of providing very high water absorbency, many of the particles shake out during preparation of the articles on high speed manufacturing machines.

One attempt to deal with the issue of shake-out is described in U.S. Patent No. 5,641,561 to Hansen et al. Hansen et al. described absorbent composites made of fibrous material (e.g. cellulosic or synthetic material) and SAP particles that are bound to the fiber via hydrogen bonding binder molecules. SAP particles are mixed with bleached kraft fluff, heated and spread out to dry. The binder adheres the SAP particles to the fibers. The dried product is then fed through a hammermill and shunted to an airlay line to produce a web containing 40% SAP particles attached to individual fibers.

U.S. Patent No. 5,593,399 to Tanzer discloses an absorbent article, namely a diaper, having two layers attached by an adhesive to provide attached zones and unattached zones to form pocket regions. Tanzer describes the use of SAP particles located within the pocket regions of the article to provide an absorbent laminate.

Another difficulty with absorbent products is that they sometimes exhibit a problem known as "gel blocking." This refers to the tendency of SAP particles, when evenly distributed in an absorbent matrix, to swell so much upon imbibing a fluid that they occlude the interstitial voids in the matrix, thereby restricting or preventing further fluid flow. The result is a tendency for leakage when a second flow of liquid occurs, since much of the SAP is inaccessible to the liquid due to the blocking effect. Thus, much of the SAP is inefficiently used.

Another problem with absorbent products is strength in that, upon exposure to a fluid, the entire substrate may become or remain wet. Because of such wetting, the strength may be diminished, leading to tearing problems.

Thus there remains a need for an improved absorbent article design that overcomes one or more of the foregoing problems associated with conventional designs.

### SUMMARY OF THE INVENTION

In one exemplary aspect, the invention provides an absorbent article comprising a barrier layer, a cover layer extending substantially parallel to the barrier layer, and a superabsorbent polymer interposed between the cover layer and the barrier layer. The superabsorbent polymer is adhered to the article in a pattern configured to distribute fluid in the absorbent article, wherein at least one portion extending essentially completely across the absorbent article is substantially devoid of the superabsorbent polymer.

According to one exemplary embodiment, the absorbent article optionally includes a tissue layer interposed between the cover layer and the barrier layer. The superabsorbent polymer is optionally provided on the tissue layer in a pattern configured to distribute fluid in the absorbent article.

In another exemplary aspect, the invention provides a method of making an absorbent article. The method includes positioning a superabsorbent polymer adjacent a barrier layer in a pattern to form at least one region including the superabsorbent polymer and at least one region substantially devoid of the superabsorbent polymer extending essentially completely across the barrier layer, thereby providing the region substantially devoid of superabsorbent polymer with greater tear resistance than the region including the superabsorbent polymer. A cover layer is attached substantially parallel to and substantially coextensive with the barrier layer, thereby interposing the superabsorbent polymer between the barrier layer and the cover layer.

In yet another exemplary embodiment, the method includes positioning a liquid comprising a superabsorbent precursor adjacent a barrier layer in a pattern to form at least one region including the superabsorbent precursor and at least one region substantially devoid of the superabsorbent precursor extending essentially completely across the barrier layer, thereby providing the region substantially devoid of superabsorbent polymer with greater tear resistance than the region including the superabsorbent polymer. The superabsorbent precursor is converted to a superabsorbent polymer, thereby forming at least one region including the superabsorbent polymer and at least one region devoid of the superabsorbent polymer. A cover layer is coupled substantially parallel to and substantially coextensive with the barrier layer, thereby interposing the superabsorbent polymer between the barrier layer and the cover layer.

### BRIEF DESCRIPTION OF THE DRAWINGS

Figure 1 is a cross-sectional view of a conventional absorbent article comprising a core incorporating a tissue having multiple folds.
Figure 2A is a cross-sectional view of an exemplary embodiment of an absorbent article, according to aspects of this invention, comprising a core incorporating a superabsorbent polymer.
Figure 2B is a cross-sectional view of another exemplary embodiment of an absorbent article comprising a core layer incorporating SAP, with the core layer being a folded sheet.
Figs. 3A-3F are top views of portions of absorbent articles in which a variety of patterns of superabsorbent polymer distribution are shown, according to exemplary embodiments of the invention.
Figure 4 is a cross-sectional view of yet another exemplary embodiment of the present invention.
Figure 5 is a top view of the absorbent article of Figure 4, showing one exemplary pattern of superabsorbent polymer application.

### DETAILED DESCRIPTION OF THE INVENTION

This invention will now be described with reference to specific embodiments selected for illustration in the drawings, wherein similar numbers indicate similar features.

The scope of this invention is defined separately in the appended claims. Also, it will be appreciated that the drawings are not rendered to any particular proportion or scale.

An absorbent article and method of making such an article are provided. More specifically, exemplary embodiments of this invention relate to an absorbent article wherein liquid superabsorbent polymer has been applied either directly to a fluid-impermeable barrier layer or to a porous core positioned adjacent the barrier layer, in any of a variety of patterns. The absorbent core may be covered with a woven or non-woven, fluid-permeable cover layer on the surface opposite that of the barrier layer. A nonlimiting example of such an absorbent article is an underpad designed to provide fluid protection for beds and other external surfaces. Other absorbent articles such as diapers, incontinence pads, and feminine hygiene pads, may also be made according to this invention.

As used herein, the terms "liquid SAP" and "liquid superabsorbent polymer" mean a liquid comprising one or more crosslinked or uncrosslinked superabsorbent polymers in dispersed or dissolved form, or a liquid comprising one or more superabsorbent precursors, or a combination of these. Superabsorbent precursors include *inter alia* oligomers and monomers that, after placement on a substrate, are subsequently converted , for example by polymerization and/or crosslinking, to a superabsorbent polymer.

Application of liquid SAP to an absorbent article provides the article with added total absorbent capacity, thereby reducing the amount of core material needed to obtain a targeted total capacity. Since less material is needed to obtain targeted total absorption capacity, a thin absorbent material may be provided by the present invention. Thus, in order to provide an "ultra-thin" absorbent core, use of the liquid superabsorbent reduces the number and/or thickness of layers needed to obtain a targeted total absorbent capacity. This type of construction affords a thinner product, desirable for comfort and/or cost savings on material consumption and handling. The liquid SAP may be applied to the core. For some applications, no core at all may be used, with SAP being only on the barrier layer of the absorbent article.

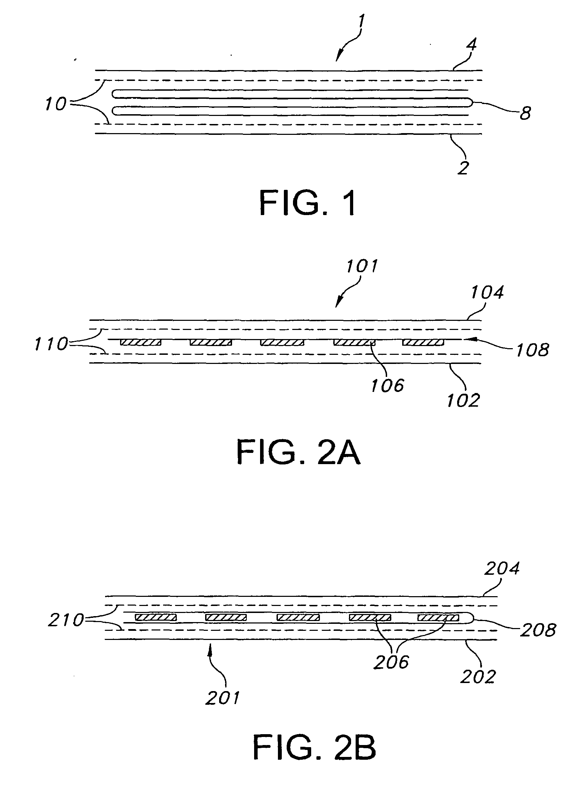

Figure 1 shows a prior art absorbent article 1 in which an absorbent core 8 is interposed between a barrier layer 2 and a cover layer 4, both of which are typically adhered to core 8 by means of adhesive 10. Core 8 comprises an absorbent substrate that is symmetrically folded upon itself such that all of the layers extend substantially parallel and substantially coextensive with each other. Typically, cover layer 4 and barrier layer 2 are adhered together in areas beyond core 8 around the perimeter (not shown), in order to provide structural integrity for the absorbent article.

Figure 2A shows, in one exemplary embodiment of the invention, an absorbent article 101, in which core 108 is a single layer of material. SAP 106 is present in a pattern on the core 108, which may be adhered with adhesive 110 to a cover layer 104 and a barrier layer 102. As used herein, when SAP is stated to be "on" or "adhered to" a core, it is to be understood that some of the SAP, having been deposited in a liquid form, may penetrate into interstices or pores of the core, which may or may not have interstices or pores depending upon the core selected.

The core 108 may be preferably made of any cellulosic material such as for example tissue or paper towel sheets, especially when a lower product cost is desired. The use of tissue is also advantageous in providing a low weight pad, and one of good wet integrity. Pulp may also be used in the core. Specifically, core 108 may comprise a single layer of defibrated pulp embossed for rapid fluid absorption and dispersion. Other types of cores may also be used. Nonlimiting examples include fibrous webs made of natural or synthetic fibers, for example, woven fabric, non-woven fabric, paper and knit fabric. Also, hydrophilic fibers such as wood pulp, cotton, wool, rayon, acetate, and vinylon, for example may be used for making core 108. An air laid composite may also be used. As discussed elsewhere, the care can also be eliminated from the absorbent article.

The barrier layer 102 may be made of any liquid impermeable material such as polyethylene, polypropylene, a copolymer thereof, polyester, and bi-component fibers. Other suitable materials include acrylic, nylon, and polyvinyl chloride. The barrier layer 102 prevents the passage of liquid and covers the underside of the absorbent article for protection against leakage.

Cover layer 106 may be formed of a woven or non-woven material, such as a substrate of thermoplastic fibers, or an apertured film. Materials used for cover layers are well known in the art and are selected from materials that are fluid permeable; that is, they allow the passage of liquid therethrough. The cover layer 106 and the barrier layer 102 are typically attached to the core 108 by means of adhesive 110, but other means may also be used in addition or instead.

One type of adhesive 110 that may be used is a hot melt pressure-sensitive adhesive. Such adhesives typically comprise one or more base polymers, one or more tackifying resins, a plasticizer, one or more waxes, and a filler and/or a pigment. Base polymers include for example polyethylene, amorphous polypropylene, ethylene-vinyl acetate copolymers, or block copolymers. Tackifying resins include for example hydrocarbon resins, rosin esters, or terpene-based resins made from such feedstocks as alpha pinene, beta pinene, limonene, dipentene, and related materials. Wax types include paraffin, microcrystalline, or synthetic waxes. Plasticizer types include for example polybutene and mineral oil. Other additives may include for example antioxidants such as sterically hindered phenols, phosphites, synergists or amides. In addition, other types of adhesive such as cold glue emulsions or cohesives may be used.

SAP 106 is positioned by applying a liquid SAP in a pattern designed to ensure that absorption of the insult will occur primarily where the liquid superabsorbent is applied. Thus, the liquid SAP permits a controlled application of the SAP especially in specific patterns, as will be discussed below. Also, by using liquid SAP, the manufacturer may be able to make a finished product on converting lines without pulp hammer mills, if that is desired.

Numerous liquid SAPs are known in the art. Examples of liquid SAPs suitable for use with this invention will now be described. In all cases, the final result is the formation of a superabsorbent polymer adhered to one or both of the barrier layer and the core.

U.S. Patent No. 4,944,963 to Dabi describes forming a terpolymer of methyl methacrylate, acrylic acid, and glycidyl methacrylate, with the terpolymer subsequently being neutralized. The resulting solution is applied to a polyester fibrous weave in an aqueous form. Excess solution is removed by vacuum, and the fiber/polyester composite is heated for 20 minutes at 120°C to create a structure of 40% fibrous weave and 60% crosslinked polymer.

U.S. Patent No. 5,853,867 to Harada describes a cationic absorbent polymer applied to a fibrous polyester weave as a monomer solution, with the monomer subsequently being polymerized on the weave by heating. Alternatively, preformed polymer may be applied. Halogenated alkyl quaternary salts of dialkylamino (meth)acrylates are effectively used for the production of the polymers, and include the halogenated alkyl quaternary salts of such monomers as N,N-dimethylaminoethyl (meth)acrylate; N,N-dimethylaminopropyl (meth)acrylate; N,N-diethylaminobutyl (meth)acrylate; N,N-diethylaminoethyl (meth)acrylate; and N,N-diethylaminopropyl (meth)acrylate; for example. As used herein, the term "(meth)acrylate" means methacrylate and/or acrylate. Such a cationic absorbent polymer may be applied in a suitable liquid form, such as for example a solution, by means of spraying or spreading to the supporting member and fixed thereto. Preferably, the fixation of the cationic absorbent polymer to the supporting member is attained by depositing a monomer or mixture of monomers in the form of an aqueous solution on the supporting member by means of impregnation, for example, and polymerizing or polycondensing the applied layer of the monomer solution.

Another suitable liquid SAP is disclosed in U.S. Pat. No. 6,417,425 to Whitmore et al. There is described a sprayable composition comprising a monomer such as acrylic acid, methacrylic acid, and/or salts of these; a crosslinker such as ethoxylated and propoxylated trimethylolpropane triacrylate derivatives such as SR-9035 and SR-492 available from Sartomer Co., Inc. of Exton, Pa.; a polymerization initiator such as 2,2'-azobis(2-(2-imidazole-2-yl))propane dihydrochloride, and solid superabsorbent particles prepared for example from acrylic acid and crosslinked with for example an unsaturated crosslinking agent such as tetraallyloxyethane, all in aqueous solution. The mixture can be sprayed onto a fibrous substrate and subsequently cured via heating or exposure to electron beam or ultraviolet radiation, to form a superabsorbent polymer matrix.

Liquid SAP applied to a core may penetrate essentially through the entire thickness thereof. In a preferred embodiment, however, the superabsorbent polymer 106 is more heavily concentrated on one surface of core 108. That surface is positioned adjacent the barrier layer 102 for the purpose of avoiding gel blocking. In other words, the side of the core 108 that contains the highest loading of the superabsorbent polymer 106 is positioned to face the barrier layer 102. Since SAP 106 swells when wet, it tends to occlude the microscopic fluid wicking channels between the pulp fibers. By positioning the side of core 108 having less (or no) SAP nearer the cover layer 104, which is adjacent the user's body, fluids pass through portions of the core 108 where there is a diminished chance of gel blocking, before the fluid finally reaches the heavier concentration of SAP near the barrier layer 102.

Figure 2B shows another exemplary embodiment of the invention, in which core 208 is a folded layer of material. SAP 206 may be incorporated on either one half of or the entire width of core 208, which is then folded. The core 208 is then interposed between cover layer 204 and barrier layer 202, with the assembly being bonded together with adhesive 210.

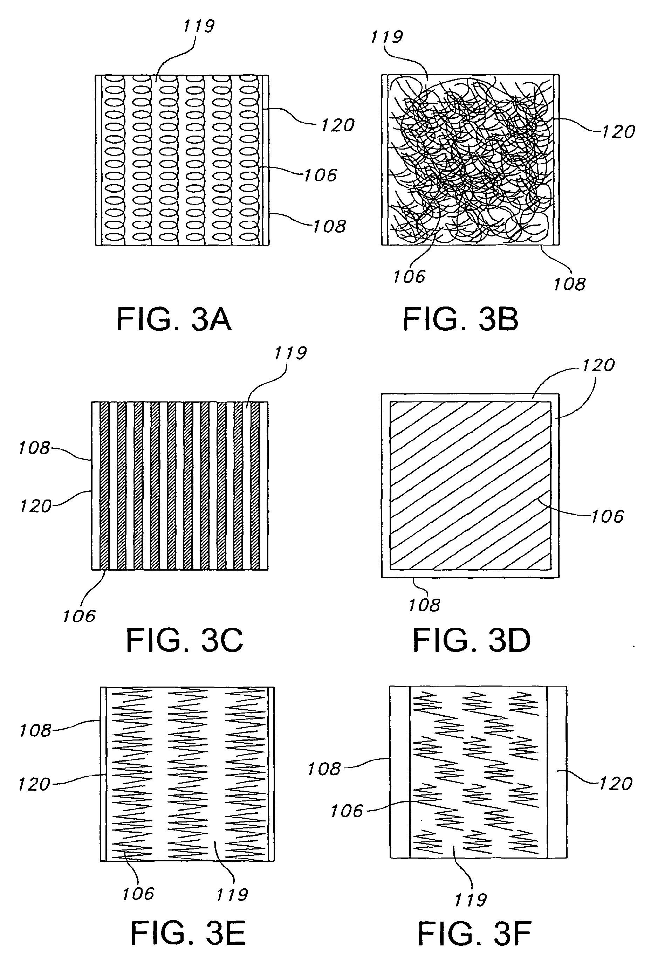

Figures 3A through 3F show top views of several possible, nonlimiting versions of core 108 corresponding to the absorbent article of Figure 2A according to exemplary embodiments of the invention, differing from each other in the pattern in which SAP 106 has been applied to core 108. Although the discussion here will generally refer to a one-layer core such as shown in Figure 2A, the discussion also applies to folded structures such as shown in Figure 2B, where the patterns may be present on one or more of the folds. Although core 108 is shown here as a square, it may instead be any other convenient shape.

Figures 3A-3F show examples of core 108, indicating that the patterns of SAP 106 may be arranged such that there exist regions 119 devoid of SAP. In all of the exemplary embodiments illustrated in Figures 3A-3F, there is at least one direction across the surface of core 108 in which tearing would require separation across regions devoid of SAP. While such a configuration is preferred, however, it is not a critical feature of the invention.

Such areas devoid of SAP, by virtue of being adjacent areas with SAP, remain drier than would be the case if there were SAP present in them. The result is that these areas retain much of their strength even when the absorbent article is wet, since the SAP imbibes most of the water. Thus the likelihood of accidental tearing of core 108 is reduced, according to an exemplary aspect of the invention. In addition, as seen in Figures 3A-3E, there may optionally be continuous SAP-free zones 120 along one or more edges of the absorbent article. Such zones provide an extra element of protection against tearing when the article is wetted but they are not required. In the case of Figure 3D, where the diagonal lines indicate a full continuous coverage of SAP 106, the only SAP-free areas are those afforded by SAP-free zone 120. Figure 3D shows continuous SAP-free zones 120 extending not only along the side borders, but along the top and bottom of core 108 as well.

The application of the liquid SAP may be performed in a variety of patterns as shown in Figs. 3A-3F. These patterns include spiral patterns 3A, melt blown patterns 3B, multi-tracked patterns 3C, full coat patterns 3D, zoned spray patterns 3E, and intermittent patterns 3F. Patterned application of liquid SAP not only provides improved wet strength integrity to the tissue core; it also provides controlled distribution of fluid. Specifically, the patterns allow fluid to be drawn by the SAP to specific regions of the absorbent article, while leaving other areas devoid of SAP in order to maintain open porosity when wet. This preserves the presence of fluid distribution channels, thereby avoiding gel blocking.

In Figure 3F, the intermittent pattern provides tear resistance diagonally, vertically, and horizontally since the non-SAP regions 119 must be crossed by a tear propagating in any of these directions.

Similarly, the melt blown pattern of Figure 3B provides tear resistance in the continuous "dry" zones 120 and in regions 119. Figure 3C has a striped pattern of SAP and non-SAP areas bordered by continuous zones 120 which provide greater tear resistance than the areas containing SAP 106.

Figure 3D shows a full coat pattern of SAP 106 on a core 108 comprising tissue incorporating a wet strength resin (not shown) to afford additional tear resistance when wet. Suitable wet strength resins are well known in the a rt, and include for example Kymene® 557H wet strength resin, available from Hercules Incorporated, Wilmington, DE. Tear resistance of core 108 in Figure 3D is further enhanced by the presence of continuous zones 120 devoid of SAP, extending along all four borders of core 108.

Figure 3E shows a core 108 with SAP 106 distributed in three zigzag patterns, separated by SAP-free areas 119 and bordered by SAP-free zones 120. Although three zigzags are shown, there may be any number of them. In addition, they need not be parallel as shown in Figure 3E, and the zigzags need not have sharply defined corners.

Figure 3F shows a core with continuous zones 120 and regions 119 devoid of SAP 106, thereby providing greater tear resistance than the patterned areas containing SAP. In this embodiment, SAP-free region 119 is continuous, with islands of SAP 106 interspersed in it.

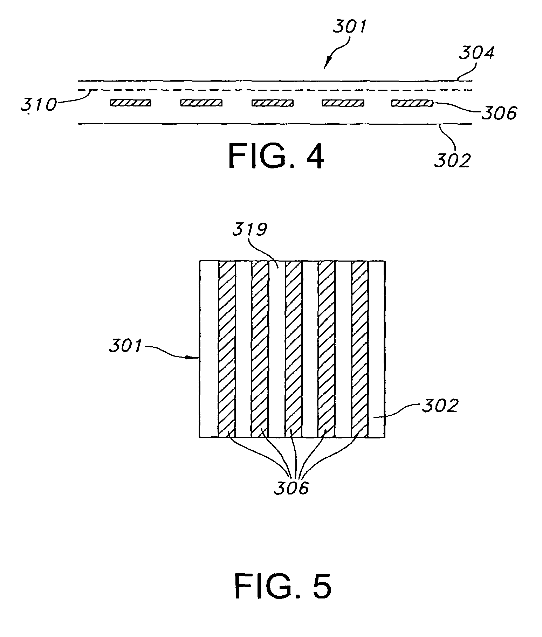

Figure 4 depicts another exemplary embodiment of the invention, showing an absorbent article 301 comprising a barrier layer 302, a cover layer 304, and SAP 306. The SAP resides directly on the barrier layer 302, which is bonded to cover layer 304 around the outer edges (not shown) of the article, using adhesive 310.

Figure 5 shows a top view of the barrier layer 302 of the absorbent article 301 of Figure 4, showing SAP 306 disposed in a striped pattern, according to one exemplary embodiment of the invention. Cover layer 304 is not shown, for clarity. Areas 319 devoid of SAP are present to afford improved fluid flow and reduced gel blocking, as described previously. By virtue of not using a core, a thinner and more comfortable structure may be obtained.

The patterns of SAP shown in Figures 3A-3F may be obtained by use of commercially available application equipment, well known to those skilled in th e art. For example, Figures 3A, 3E, and 3F represent patterns of SAP distribution that might typically be obtained using a spiral-spray nozzle head. The pattern of Figure 3A may be achieved by application in a "controlled fiberization" configuration using a low air pressure spray head. The patterns of Figures 3E and 3F may typically use higher air pressure, with the pattern of Figure 3E being continuous and that of Figure 3F being intermittent in terms of SAP distribution. The latter may typically be produced by turning the nozzles on and off during the spraying process.

Figures 3C and 3D show patterns that might typically be achieved by applying liquid SAP with a slot-coat application nozzle, with Figure 3D being achieved by use of a single wide track and Figure 3C with multiple tracks. The pattern of Figure 3B may typically be achieved via use of a melt-blown application nozzle, which produces noncontinuous, sporadic deposition of the SAP.

While preferred embodiments of the invention have been shown and described herein, it will be understood that such embodiments are provided by way of example only.

For example, according to exemplary aspects of this invention, an absorbent article is optionally formed by application of SAP directly to a barrier layer of the absorbent article (see, for example, Figures 4 and 5). More specifically, SAP is optionally applied directly to a barrier layer to supplement a core interposed between a barrier layer and a cover layer or to eliminate such a core.

According to one exemplary embodiment so configured, SAP is applied directly to a film barrier. The film barrier is optionally em bossed film and may be embossed to have a "male" side opposite a "female" defining cavities. If such an embossed film barrier is utilized, the SAP is preferably applied to the female side of the embossed film so as to at least partially fill the cavities. The SAP is optionally applied to such a film barrier in patterns to facilitate fluid distribution. To do so, a liquid SAP may be applied in a manner set forth previously.

## Claims

1. An absorbent article comprising:
a barrier layer;
a cover layer extending substantially parallel to said barrier layer; and
a superabsorbent polymer interposed between said cover layer and said barrier layer, said superabsorbent polymer being adhered to said article in a pattern configured to distribute fluid in said absorbent article, wherein at least one portion of said absorbent article extending essentially completely across said absorbent article is substantially devoid of said superabsorbent polymer.

2. The absorbent article of claim 1 further comprising a core interposed between said cover layer and said barrier layer, wherein said superabsorbent polymer is applied to said core or said barrier layer in said pattern.

3. The absorbent article of claim 1 wherein said pattern is configured to increase resistance of said absorbent article to tearing, said at least one portion of said absorbent article extending essentially completely across said absorbent article being more resistant to tearing than at least one other portion of said absorbent article.

4. The absorbent article of claim 1 further comprising a core interposed between said cover layer and said barrier layer, wherein said core comprises at least one of cellulose and cellulose acetate.

5. The absorbent article of claim 4 wherein said core is selected from the group consisting of tissue, air laid composite, and paper towel sheet.

6. The absorbent article of claim 1 wherein said barrier layer comprises a material selected from the group consisting of polyethylene, polypropylene, copolymers of polyethylene and polypropylene, polyester, and bi-component fibers.

7. The absorbent article of claim 1 wherein said cover layer comprises one or both of a non-woven material and an apertured film.

8. The absorbent article of claim 1 wherein said pattern forms at least one region including said superabsorbent polymer and at least one continuous zone that is substantially devoid of said superabsorbent polymer, said continuous zone having greater tear resistance than said region including said superabsorbent polymer.

9. The absorbent article of claim 8 wherein said pattern is selected from the group consisting of a spiral pattern, a melt blown pattern, a multi-tracked pattern, a full coat pattern, a zoned spray pattern, and an intermittent pattern.

10. The absorbent article of claim 1 wherein said superabsorbent polymer is formed from one or more of a polymer in liquid form and a polymer formed by conversion of a superabsorbent precursor, said superabsorbent precursor comprising one or both of a monomer and an oligomer.

11. A method of making an absorbent article comprising the steps of:
a) positioning a superabsorbent polymer adjacent a barrier layer in a pattern to form at least one region including the superabsorbent polymer and at least one region substantially devoid of said superabsorbent polymer extending essentially completely across the barrier layer, thereby providing the region substantially devoid of superabsorbent polymer with greater tear resistance than the region including the superabsorbent polymer; and
b) attaching a cover layer substantially parallel to and substantially coextensive with said barrier layer, thereby interposing said superabsorbent polymer between the barrier layer and the cover layer.

12. The method of claim 11 further comprising the step of interposing a core between the barrier layer and the cover layer, wherein said positioning step comprises applying the superabsorbent polymer to the core.

13. A method of making an absorbent article comprising the steps of:
a) positioning a liquid comprising a superabsorbent precursor adjacent a barrier layer in a pattern to form at least one region including the superabsorbent precursor and at least one region substantially devoid of the superabsorbent precursor extending essentially completely across the barrier layer, thereby providing the region substantially devoid of superabsorbent polymer with greater tear resistance than the region including the superabsorbent polymer;
b) converting the superabsorbent precursor to a superabsorbent polymer, thereby forming at least one region including the superabsorbent polymer and at least one region devoid of the superabsorbent polymer; and
c) coupling a cover layer to the barrier layer, thereby interposing the superabsorbent polymer between the barrier layer and the cover layer.

14. The method of claim 13 further comprising the step of interposing a core between the barrier layer and the cover layer, wherein said positioning step comprises applying the liquid to the core.

15. An absorbent article comprising:
a barrier layer;
a cover layer extending substantially parallel to said barrier layer;
an absorbent layer interposed between said cover layer and said barrier layer; and
a superabsorbent polymer applied in liquid form to said absorbent layer, said superabsorbent polymer being applied in a pattern configured to distribute fluid in said absorbent article, wherein portions of said absorbent layer are at least partially coated with said superabsorbent polymer and other portions of said absorbent layer are substantially free of said superabsorbent polymer.

16. An absorbent underpad comprising:
a barrier layer;
a cover layer extending substantially parallel to said barrier layer;
a tissue layer interposed between said cover layer and said barrier layer; and
a superabsorbent polymer applied in liquid form to said absorbent layer, said superabsorbent polymer being applied in a pattern configured to distribute fluid in said absorbent article, wherein portions of said absorbent layer are at least partially coated with said superabsorbent polymer and other portions of said absorbent layer are substantially free of said superabsorbent polymer.

## Patentansprüche

1. Absorbierender Gegenstand, umfassend:
eine Barrierelage,
eine Abdecklage, welche sich im Wesentlichen parallel zu der Barrierelage erstreckt, und
ein superabsorbierendes Polymer, welches zwischen der Abdecklage und der Barrierelage angeordnet ist, wobei das superabsorbierende Polymer an dem Gegenstand in einem Muster angebracht ist, welches ausgelegt ist um Fluid in dem absorbierenden Gegenstand zu verteilen, wobei mindestens ein Abschnitt des absorbierenden Gegenstands, der sich im Wesentlichen vollständig über den absorbierenden Gegenstand erstreckt, im Wesentlichen frei von dem superabsorbierenden Polymer ist.

2. Absorbierender Gegenstand nach Anspruch 1, weiterhin umfassend einen zwischen der Abdecklage und der Barrierelage angeordneten Kern, wobei das superabsorbierende Polymer in dem Muster auf dem Kern oder der Barrierelage aufgebracht ist.

3. Absorbierender Gegenstand nach Anspruch 1, wobei das Muster ausgelegt ist um die Beständigkeit des absorbierenden Gegenstands gegen Zerreißen zu erhöhen, wobei der mindestens eine Abschnitt des absorbierenden Gegenstands, der sich im Wesentlichen vollständig über den absorbierenden Gegenstand erstreckt, beständiger gegen Zerreißen ist als mindestens ein anderer Abschnitt des absorbierenden Gegenstands.

4. Absorbierender Gegenstand nach Anspruch 1, weiterhin umfassend einen zwischen der Abdecklage und der Barrierelage angeordneten Kern, wobei der Kern Cellulose und/oder Celluloseacetat umfasst.

5. Absorbierender Gegenstand nach Anspruch 4, wobei der Kern ausgewählt ist aus der Gruppe, bestehend aus Tissue, einem Airlaid-Verbundstoff, und einer Papierhandtuchlage.

6. Absorbierender Gegenstand nach Anspruch 1, wobei die Barrierelage ein Material umfasst, ausgewählt aus der Gruppe, bestehend aus Polyethylen, Polypropylen, Copolymeren von Polyethylen und Polypropylen, Polyester, und Bikomponentenfasern.

7. Absorbierender Gegenstand nach Anspruch 1, wobei die Abdecklage ein Vliesmaterial und/oder eine geöffnete Folie umfasst.

8. Absorbierender Gegenstand nach Anspruch 1, wobei das Muster mindestens eine Region bildet, welche das superabsorbierende Polymer beinhaltet, und mindestens eine kontinuierliche Zone, die im Wesentlichen frei von dem superabsorbierenden Polymer ist, wobei die kontinuierliche Zone eine größere Beständigkeit gegen Zerreißen aufweist als die Region, welche das superabsorbierende Polymer beinhaltet.

9. Absorbierender Gegenstand nach Anspruch 8, wobei das Muster ausgewählt ist aus der Gruppe, bestehend aus einem Spiralmuster, einem Schmelzblasmuster, einem Mehrbahnenmuster, einem Vollauftragmuster, einem Zonensprühmuster und einem intermittierenden Muster.

10. Absorbierender Gegenstand nach Anspruch 1,wobei das superabsorbierende Polymer aus einem Polymer in flüssiger Form und/oder einem durch Umsetzung eines superabsorbierenden Vorläufers gebildeten Polymer gebildet ist, wobei der superabsorbierende Vorläufer ein Monomer und/oder ein Oligomer umfasst.

11. Verfahren zur Herstellung eines absorbierenden Gegenstands, umfassend die Schritte:
a) Positionieren eines superabsorbierenden Polymers benachbart zu einer Barrierelage in einem Muster, so dass mindestens eine Region gebildet wird, welche das superabsorbierende Polymer beinhaltet, und mindestens eine Region, welche im Wesentlichen frei von dem superabsorbierenden Polymer ist und sich im Wesentlichen vollständig über die Barrierelage erstreckt, wodurch der Region, welche im Wesentlichen frei von dem superabsorbierenden Polymer ist, eine größere Beständigkeit gegen Zerreißen vermittelt wird, als der Region, welche das superabsorbierende Polymer beinhaltet, und
b) Anbringen einer Abdecklage im Wesentlichen parallel zu der Barrierelage, und die sich im Wesentlich gleich wie die Barrierelage erstreckt, wodurch das superabsorbierende Polymer zwischen der Barrierelage und der Abdecklage angeordnet wird.

12. Verfahren nach Anspruch 11, weiterhin umfassend den Schritt des Anordnens eines Kerns zwischen der Barrierelage und der Abdecklage, wobei der Schritt des Positionierens das Aufbringen des superabsorbierenden Polymers auf den Kern umfasst.

13. Verfahren zur Herstellung eines absorbierenden Gegenstands, umfassend die Schritte:
a) Positionieren einer Flüssigkeit, umfassend einen superabsorbierenden Vorläufer, benachbart zu einer Barrierelage in einem Muster, so dass mindestens eine Region gebildet wird, welche den superabsorbierenden Vorläufer beinhaltet, und mindestens eine Region, welche im Wesentlichen frei von dem superabsorbierenden Vorläufer ist und sich im Wesentlichen vollständig über die Barrierelage erstreckt, wodurch der Region, welche im Wesentlichen frei von dem superabsorbierenden Polymer ist, eine größere Beständigkeit gegen Zerreißen vermittelt wird, als der Region, welche das superabsorbierende Polymer beinhaltet,
b) Umsetzen des superabsorbierenden Vorläufers zu einem superabsorbierenden Polymer, wodurch mindestens eine Region, welche das superabsorbierende Polymer beinhaltet, und mindestens eine Region, welche frei von dem superabsorbierenden Polymer ist, gebildet wird, und
c) Koppeln einer Abdecklage an die Barrierelage, wodurch das superabsorbierende Polymer zwischen der Barrierelage und der Abdecklage angeordnet wird.

14. Verfahren nach Anspruch 13, weiterhin umfassend den Schritt des Anordnens eines Kerns zwischen der Barrierelage und der Abdecklage, wobei der Schritt des Positionierens das Aufbringen der Flüssigkeit auf den Kern umfasst.

15. Absorbierender Gegenstand, umfassend:
eine Barrierelage,
eine Abdecklage, welche sich im Wesentlichen parallel zu der Barrierelage erstreckt,
eine absorbierende Lage, welche zwischen der Abdecklage und der Barrierelage angeordnet ist, und
ein superabsorbierendes Polymer, welches in flüssiger Form auf die absorbierende Lage aufgebracht worden ist, wobei das superabsorbierende Polymer in einem Muster angebracht ist, welches ausgelegt ist um Fluid in dem absorbierenden Gegenstand zu verteilen, wobei Abschnitte der absorbierenden Lage mindestens teilweise mit dem superabsorbierenden Polymer beschichtet sind und andere Abschnitte der absorbierenden Lage im Wesentlichen frei von dem superabsorbierenden Polymer sind.

16. Absorbierende Unterlage, umfassend:
eine Barrierelage,
eine Abdecklage, welche sich im Wesentlichen parallel zu der Barrierelage erstreckt,
eine Tissue- Lage, welche zwischen der Abdecklage und der Barrierelage angeordnet ist, und
ein superabsorbierendes Polymer, welches in flüssiger Form auf die absorbierende Lage aufgebracht worden ist, wobei das superabsorbierende Polymer in einem Muster angebracht ist, welches ausgelegt ist um Fluid in dem absorbierenden Gegenstand zu verteilen, wobei Abschnitte der absorbierenden Lage mindestens teilweise mit dem superabsorbierenden Polymer beschichtet sind und andere Abschnitte der absorbierenden Lage im Wesentlichen frei von dem superabsorbierenden Polymer sind.

## Revendications

1. Article absorbant comprenant :
une couche barrière ;
une couche de couverture s'étendant sensiblement parallèlement à ladite couche barrière ; et
un polymère superabsorbant interposé entre ladite couche de couverture et ladite couche barrière, ledit polymère superabsorbant étant collé au dit article selon un motif configuré pour distribuer un fluide dans ledit article absorbant, dans lequel au moins une partie dudit article absorbant s'étendant sensiblement complètement à travers ledit article absorbant est substantiellement dépourvue dudit polymère superabsorbant.

2. Article absorbant selon la revendication 1, comprenant en outre un coeur interposé entre ladite couche de couverture et ladite couche barrière, dans lequel ledit polymère superabsorbant est appliqué au dit coeur ou à ladite couche barrière selon ledit motif.

3. Article absorbant selon la revendication 1, dans lequel ledit motif est configuré pour faire augmenter la résistance dudit article absorbant à la déchirure, ladite au moins une partie dudit article absorbant s'étendant sensiblement complètement à travers ledit article absorbant étant plus résistante à la déchirure qu'au moins une autre partie dudit article absorbant.

4. Article absorbant selon la revendication 1, comprenant en outre un coeur interposé entre ladite couche de couverture et ladite couche barrière, dans lequel ledit coeur comprend au moins l'un de la cellulose et de l'acétate de cellulose.

5. Article absorbant selon la revendication 4, dans lequel ledit coeur est choisi dans le groupe constitué par un tissu, un composite air-laid, et une serviette en papier.

6. Article absorbant selon la revendication 1, dans lequel ladite couche barrière comprend un matériau choisi dans le groupe constitué par le polyéthylène, le polypropylène, les copolymères de polyéthylène et de polypropylène, le polyester, et les fibres à deux composants.

7. Article absorbant selon la revendication 1, dans lequel ladite couche de couverture comprend l'un ou les deux d'un matériau non tissé et d'un film à ouvertures.

8. Article absorbant selon la revendication 1, dans lequel ledit motif forme au moins une région comprenant ledit polymère superabsorbant et au moins une zone continue qui est substantiellement dépourvue dudit polymère superabsorbant, ladite zone continue ayant une résistance à la déchirure supérieure à celle de ladite région comprenant ledit polymère superabsorbant.

9. Article absorbant selon la revendication 8, dans lequel ledit motif est choisi dans le groupe constitué par un motif en spirale, un motif de fusion soufflage, un motif à pistes multiples, un motif de couche complète, un motif de pulvérisation en zones, et un motif intermittent.

10. Article absorbant selon la revendication 1, dans lequel ledit polymère superabsorbant est formé à partir d'un ou de plus d'un polymère sous une forme liquide et d'un polymère formé par conversion d'un précurseur superabsorbant, ledit précurseur superabsorbant comprenant l'un ou les deux d'un monomère et d'un oligomère.

11. Procédé de fabrication d'un article absorbant comprenant les étapes consistant à :
a) positionner un polymère superabsorbant adjacent à une couche barrière selon un motif pour former au moins une région comprenant le polymère superabsorbant et au moins une région substantiellement dépourvue dudit polymère superabsorbant s'étendant sensiblement complètement à travers la couche barrière, dotant ainsi la région substantiellement dépourvue du polymère superabsorbant d'une plus grande résistance à la déchirure que la région comprenant le polymère superabsorbant ; et
b) fixer une couche de couverture sensiblement parallèle à et sensiblement de même étendue que ladite couche barrière, interposant ainsi ledit polymère superabsorbant entre la couche barrière et la couche de couverture.

12. Procédé selon la revendication 11, comprenant en outre l'étape consistant à interposer un coeur entre la couche barrière et la couche de couverture, dans lequel ladite étape de positionnement comprend l'application du polymère superabsorbant au coeur.

13. Procédé de fabrication d'un article absorbant comprenant les étapes consistant à :
a) positionner un liquide comprenant un précurseur superabsorbant adjacent à une couche barrière selon un motif pour former au moins une région comprenant le précurseur superabsorbant et au moins une région substantiellement dépourvue du précurseur superabsorbant s'étendant sensiblement complètement à travers la couche barrière, dotant ainsi la région substantiellement dépourvue du polymère superabsorbant d'une plus grande résistance à la déchirure que la région comprenant le polymère superabsorbant ;
b) convertir le précurseur superabsorbant en un polymère superabsorbant, formant ainsi au moins une région comprenant le polymère superabsorbant et au moins une région dépourvue du polymère superabsorbant ; et
c) coupler une couche de couverture à la couche barrière, interposant ainsi le polymère superabsorbant entre la couche barrière et la couche de couverture.

14. Procédé selon la revendication 13, comprenant en outre l'étape consistant à interposer un coeur entre la couche barrière et la couche de couverture, dans lequel ladite étape de positionnement comprend l'application du liquide au coeur.

15. Article absorbant comprenant :
une couche barrière ;
une couche de couverture s'étendant sensiblement parallèlement à ladite couche barrière ;
une couche absorbante interposée entre ladite couche de couverture et ladite couche barrière ; et
un polymère superabsorbant appliqué sous une forme liquide à ladite couche absorbante, ledit polymère superabsorbant étant appliqué selon un motif configuré pour distribuer un fluide dans ledit article absorbant, dans lequel des parties de ladite couche absorbante sont au moins partiellement revêtues dudit polymère superabsorbant et d'autres parties de ladite couche absorbante sont substantiellement dépourvues dudit polymère superabsorbant.

16. Protège drap absorbant comprenant :
une couche barrière ;
une couche de couverture s'étendant sensiblement parallèlement à ladite couche barrière ;
une couche de tissu interposée entre ladite couche de couverture et ladite couche barrière ; et
un polymère superabsorbant appliqué sous une forme liquide à ladite couche absorbante, ledit polymère superabsorbant étant appliqué selon un motif configuré pour distribuer le fluide dans ledit article absorbant, dans lequel des parties de ladite couche absorbante sont au moins partiellement revêtues dudit polymère superabsorbant et d'autres parties de ladite couche absorbante sont substantiellement dépourvues dudit polymère superabsorbant.
